# EUROPÄISCHE PATENTSCHRIFT

(11) **EP 1 813 598 B1**
(45) Veröffentlichungstag und Bekanntmachung des Hinweises auf die Patenterteilung: **20.10.2010**
(21) Anmeldenummer: 07000739.8
(22) Anmeldetag: 16.01.2007
(51) Int. Cl.: C07C 209/78, C07C 211/50

(54) **Verfahren zur Herstellung von Di- und Polyaminen der Diphenylmethanreihe**
Method for producing di- and polyamines of the diphenylmethane series
Procédé destiné à la fabrication de di- et polyamines de la série diphénylméthane

(30) Priorität: 28.01.2006 DE 102006004047
(43) Veröffentlichungstag der Anmeldung: 01.08.2007
(73) Patentinhaber: Bayer MaterialScience AG, 51368 Leverkusen (DE)
(72) Erfinder: Pohl, Fritz, Dr., 25541 Brunsbüttel (DE); Bolton, Jeffrey, Dr., 40489 Düsseldorf (DE); Adamson, Richard, 42799 Leichlingen (DE); Müller, Heinz-Herbert, Dr., 47809 Krefeld (DE); Uchdorf, Rudolf, 200001 Shangai (CN)

(56) Entgegenhaltungen:
- EP-A2- 0 509 309
- WO-A-01/58847
- GB-A- 1 180 795
- US-A- 4 792 624

## Beschreibung

Die Erfindung betrifft ein Verfahren zur Herstellung von Di- und Polyaminen der Diphenylmethanreihe (MDA) durch Umsetzung von Anilin und Formaldehyd in Gegenwart eines sauren Katalysators nach Anspruch 1 bei dem das eingesetzte Anilin weniger als 3 Gew.-% an MDA, bezogen auf das Gewicht des eingesetzten Anilins, enthält.

Unter Di- und Polyaminen der Diphenylmethanreihe (MDA) werden Amine und Gemische von Aminen folgenden Typs verstanden: 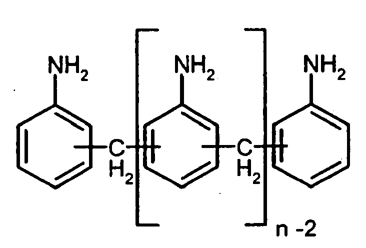 wobei n für eine natürliche Zahl ≥ 2 steht.

Für Verbindungen und Verbindungsgemische mit n = 2 sind dabei auch die Bezeichnung Monomeres MDA ( MMDA ) und für Verbindungen und Verbindungsgemische mit n > 2 auch die Bezeichnung Polymeres MDA ( PMDA ) üblich. Verbindungsgemische, in denen Verbindungen mit n = 2 und n > 2 nebeneinander vorkommen, werden üblicherweise vereinfachend unter dem Begriff MDA ( Di- und Polyamine der Diphenylmethanreihe ) zusammengefasst.

Die kontinuierliche, diskontinuierliche oder halbkontinuierliche Herstellung von Di- und Polyaminen der Diphenylmethanreihe ist in zahlreichen Publikationen und Patenten beschrieben (beispielsweise H.J. Twitchett, Chem. Soc. Rev. 3(2), 209 (1974); M.V. Moore in : Kirk-Othmer Encycl. Chem. Technol., 3rd Ed., New York, 2, 338-348 (1978); EP-A-31 423; EP-B-1 167 343; EP-A-1 403 242; EP 934 922 B1).

Üblicherweise erfolgt bei den technisch angewandten Verfahren die Herstellung von MDA durch Umsetzung von Anilin und Formaldehyd in Anwesenheit saurer Katalysatoren, wobei zum Ende des Prozesses der saure Katalysator üblicherweise durch Zugabe einer Base neutralisiert, das Reaktionsgemisch in eine organische und eine wässrige Phase getrennt und die organische Phase den abschließenden Aufarbeitungsschritten, wie beispielsweise der destillativen Entfernung von überschüssigem Anilin, zugeführt wird (US-A-5,310,769; DE-A-198 04 918; JP-A-2004026753).

Allen in der Literatur beschriebenen Verfahren zur Herstellung von MDA durch Umsetzung von Anilin und Formaldehyd in Gegenwart eines sauren Katalysators ist gemeinsam, dass bei der Umsetzung Chromophore gebildet werden, die das erzeugte MDA verfärben. Diese Verfärbungen werden bei der Neutralisation des sauren Katalysators und der Entfernung des bei der Umsetzung im Überschuss eingesetzten Anilins nicht oder nur unzureichend vermindert bzw. entfernt und führen bei der anschließenden Phosgenierung des MDA zu den entsprechenden Di- und Polyisocyanaten und deren anschließenden Aufarbeitung (Abtrennung des Lösungsmittels, Abtrennung von monomerem MDI) oft zu dunkel gefärbten Produkten, die wiederum gelblich verfärbte Polyurethanschäume oder andere verfärbte Polyurethan (PUR)-Materialien ergeben. Obwohl die Eigenfarbe der Di- und Polyisocyanate die mechanischen Eigenschaften der daraus hergestellten Polyurethane nicht negativ beeinflusst, sind helle Produkte wegen deren guten Variabilität im Produktionsprozess des Verarbeiters, z.B. hinsichtlich Durchscheinen durch dünne Deckschichten und farbliche Gestaltungsmöglichkeiten, bevorzugt.

Es hat nicht an Versuchen gefehlt, die Verfärbungen des MDA zu vermindern.

In EP 1 270 544 B1 wird ein Verfahren zur Herstellung von MDA unter Minimierung des Gehalts an unerwünschten Nebenprodukten durch die Umsetzung von Anilin mit Formaldehyd in Gegenwart saurer Katalysatoren beschrieben, dadurch gekennzeichnet, dass man in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50% der gesamt einzuspeisenden Formaldehydmenge die Reaktionsmischung auf eine Temperatur von größer 75°C temperiert. Reklamiert wird speziell die Minimierung des Gehalts an N-Methyl-MDA, dessen Verminderung im MDA gemäß der Lehre des EP 1 270 544 B1 bei einer anschließenden Phosgenierung zu einem Roh-MDI einer helleren Farbe führen soll.

Die Verbesserung der Farbwerte durch eine Verringerung des Gehalts an N-Methyl-MDA im erzeugten MDA über eine spezielle Formaldehydspeisung liegt auch folgenden Verfahren zugrunde.

DD-A-295 628 beschreibt für ein diskontinuierliches Verfahren die Zugabe des Formaldehydes in zwei Schritten während der Kondensationsstufe, wobei in der ersten Zugabe die Hauptmenge des Formaldehydes bei niederer Temperatur erfolgt und die zweite Zugabe des restlichen Formaldehyds bei gleicher oder höherer Temperatur erfolgt.

EP-A-451 442 und DD-A-238 042 offenbaren für ein kontinuierliches Verfahren die Zugabe von Formaldehyd über mehrere Verfahrensstufen.

Zur Verbesserung der Farbwerte lehrt US-A-5 286 760 neben der Minimierung des Gehalts an N-Methyl-MDA zusätzlich die Minimierung der Nebenkomponenten Acridan und Acridin. US-A-5 286 760 modifiziert jedoch nicht die Formaldehydspeisung, sondern die der primären Umsetzung des Formaldehyds mit Anilin folgenden Umlagerungen. US-A-5 286 760 beschreibt für eine kontinuierliche MDA-Herstellung eine Teilneutralisierung des Reaktionsgemisches zwischen der Kondensationsstufe von zwei Molekülen Anilin und einem Molekül Formaldehyd und der anschließenden Umlagerung der intermediär gebildeten Aminobenzylamine, abgekürzt ABA, zum MDA.

Vor allem in die Umlagerungen greift ebenfalls US-A-5 310 769 ein. US-A-5 310 769 beschreibt einen Prozess zur Herstellung von Polyaminen der Diphenylmethanreihe durch Kondensation von Anilin mit Formalaldehyd, nachfolgender Reaktion in Gegenwart eines sauren Katalysators, Neutralisation des sauren Katalysators nach vollständiger Reaktion und Reinigung des resultierenden Di- und Polyamingemischs durch Abdestillation des überschüssigen aromatischen Amins dadurch gekennzeichnet, dass in einer bevorzugten Variante
a) Anilin mit Formaldehyd in einem Molverhältnis von 1,5:1 bis 10:1 bei Temperaturen zwischen 10 und 150°C umgesetzt wird,
b) danach dem Reaktionsgemisch ein saurer Katalysator im Molverhältnis Anilin zu saurem Katalysator von 2:1 bis 100:1 bei Temperaturen zwischen 10 und 150°C zugegeben wird, dabei das bei der Kondensationsreaktion entstandene Wasser entweder vor oder nach Schritt b) abgetrennt wird,
c) anschließend die Temperatur der in Schritt b) erhaltene Mischung um mindestens 40°C innerhalb von 15 Minuten erhöht und dann weiter auf die Endtemperatur zwischen 105 und 200°C aufgeheizt und für 10 bis 300 Minuten nach der Temperaturerhöhung gehalten wird.

US-A-5 310 769 lehrt, dass durch die besondere Temperaturführung während der Kondensations- und Umlagerungsschritte ein Di- und Polyamingemisch der Diphenylmethanreihe erhalten wird, dessen nachfolgende Phosgenierung besonders helle Polyurethanschäume zugänglich macht.

Di- und Polyamingemische der Diphenylmethanreihe, deren nachfolgende Phosgenierung zu Polyisocyanaten stark reduzierter Färbung führt, werden gemäß US-A-4 792 624 auch bei Anwendung eines Verfahrens erhalten, das dadurch gekennzeichnet ist, dass
a) schnell fließende Ströme von wässrigem Anilinhydrochlorid und wässrigem Formaldehyd in einem Verhältnis von 1,6 bis 8 Molen Anilin pro Mol Formaldehyd am Einlauf eines Rohrreaktors intensiv durchmischt werden, dadurch sofort eine Aminobenzylamine enthaltende Mischung erzeugt wird,
b) die gemäß a) erzeugte Mischung nachfolgend über eine gekühlte Reaktionszone geführt wird, in der der Gehalt an Aminobenzylaminen in der Mischung mindestens auf 30 Gew.-% ansteigt,
c) das Reaktionsgemisch der gekühlten Reaktionszone in dem Maß entnommen wird, wie Reaktionsmischung aus Schritt a) nachfließt,
d) das Reaktionsgemisch der gekühlte Reaktionszone dann durch eine Umlagerungszone mit Temperaturen von 60° bis 200°C geführt wird, dabei das Polyamin der Diphenylmethanreihe gebildet wird,
e) das Reaktionsgemisch der Umlagerungszone in dem Maß entnommen wird, wie Reaktionsmischung in die Umlagerungszone gespeist wird,
f) das Reaktionsgemisch aus der Umlagerungszone kontinuierlich einer Neutralisationszone, in der die sauren Komponenten neutralisiert werden, zugeführt wird, dann Anilin und Wasser von dem Reaktionsgemisch abgetrennt werden, so ein Anilin-freies Polyamin der Diphenylmethanreihe erhalten wird,
g) das Polyamingemisch in dem Maß Schritt f) entnommen wird, wie Reaktionsmischung in die Neutralisations- bzw. Destillationsstufe eingespeist wird,
h) die Hauptmenge des gewonnenen Polyamingemischs in einen Lagertank ausgeschleust wird, ein Teilstrom des Polyamingemischs im Umfang von 1 bis 40 Gew.-%, bezogen auf die vereinigten anfänglichen Gewichte der von in Schritt a) eingespeisten Anilin-, Anilinhydrochlorid- und Formaldehydmengen, jedoch auf Schritt b) zurückgeführt und bei laufendem Prozess a) bis h) erneut durch die Schritte b) bis h) geschleust wird.

Gemäß US-A-4 792 624 ist es zur Erzielung einer maximalen Farbwertverbesserung dabei wesentlich, dass das in den Prozess eingesetzte überschüssige Anilin von dem MDA abgetrennt wird, bevor das MDA den Benzylaminen zugesetzt wird. Darüber hinaus wird nach der Lehre von US-A-4 792 624 die verbesserte Färbung nur erreicht, wenn das rückgeführte Polyamin dort zugesetzt wird, wo Aminobenzylamine vorhanden sind, und nicht in der Stufe, in der Anilin und Formalaldehyd zuerst reagieren.

Allen zitierten und in der Literatur beschriebenen Verfahren zur Herstellung von Di-und Polyaminen der Diphenylmethanreihe durch Umsetzung von Anilin mit Formalin in Gegenwart saurer Katalysatoren ist gemeinsam, dass sie durch Modifikation einzelner Verfahrensparameter, z.B. der Komponentendosierung, der Konzentration an saurem Katalysator, der Temperaturführung oder auch der Produktzusammensetzung bei den Umlagerungen, jeweils Verbesserungen bei der Färbung der erzeugten Di- und Polyamingemische bzw. der aus ihnen erzeugten Isocyanate und Polyurethane erreichen. Dennoch besteht weiterhin ein Bedarf an neuen Verfahren mit noch weitergehenden Farbverbesserungen.

Diese Aufgabe konnte überraschenderweise dadurch gelöst werden, dass bei der Herstellung der Di- und Polyamine der Diphenylmethanreihe durch Umsetzung von Anilin und Formaldehyd in Gegenwart saurer Katalysatoren ein Anilin eingesetzt wird, das weniger als 3 Gew.-%, bevorzugt 0,001 bis 3 Gew.-%, besonders bevorzugt 0,01 bis 1 Gew.-% Di- und Polyamine der Diphenylmethanreihe, bezogen auf das Gewicht des eingesetzten Anilins, enthält.

In dem erfindungsgemäßen Verfahren zur Herstellung von Di- und Polyaminen der Diphenylmethanreihe durch Umsetzung von Anilin und Formaldehyd in Gegenwart eines sauren Katalysators enthält das eingesetzte Anilin weniger als 3 Gew.-%, bevorzugt 0,001 bis 3 Gew.-%, besonders bevorzugt 0,01 bis 1 Gew.-% Di- und Polyamine der Diphenylmethanreihe, bezogen auf das Gewicht des eingesetzten Anilins.

Der erfindungsgemäße Einsatz von Anilin enthaltend weniger als 3 Gew.-%, bevorzugt 0,001 bis 3 Gew.-%, besonders bevorzugt 0,01 bis 1 Gew.-% Di- und Polyamine der Diphenylmethanreihe ist insofern von besonderer Bedeutung, dass die Umsetzung von Anilin mit Formaldehyd in Gegenwart saurer Katalysatoren zur Einstellung des gewünschten Gehalts an Diaminen sowie zur Wahrung der Handhabbarkeit der Reaktionsgemische stets mit einem Anilinüberschuss durchgeführt wird. Dieser Anilinüberschuss muss bei der Aufbereitung der Di- und Polyamingemische abgetrennt und zur Wahrung der äußeren Massenbilanz wieder auf die Reaktionsstufen des Verfahrens zurückgeführt werden.

Die Abtrennung des Anilinüberschusses aus dem bei der Umsetzung von Anilin und Formaldehyd erhaltenen Reaktionsgemisches geschieht üblicherweise destillativ, wobei ebenfalls das dem Polyamingemisch noch anhaftende Wasser abgetrennt wird. Dabei lässt sich ein weitgehend MDAfreies und wasserfreies Anilin nur unter einem erheblichen Aufwand gewinnen.

Überraschenderweise wurde zusätzlich gefunden, dass dieser Aufwand weitgehend entfallen kann, wenn die bei der Umsetzung von Anilin mit Formalin in Gegenwart saurer Katalysatoren mit nachfolgender Neutralisation und Separation und Aufbereitung der organischen Phase anfallenden Abwässer nicht wie gemäß dem Stand der Technik üblich ( JP 2004026753 ) mit einem zusätzlich eingesetzten hydrophoben Lösungsmittel extrahiert werden, sondern zur Reinigung der Abwässer die Abwässer mit den nicht weiter aufbereiteten Kondensaten aus der Anilinabtrennung vermischt werden und anschließend eine Phasentrennung durchgeführt wird. Die dabei erhaltene organische Phase kann dann wie oben beschrieben als MDA-beladenes Anilin, zur Wahrung der vorteilhaft niedrigen MDA-Gehalte ggf. unter Zuspeisung frisch eingesetzten Anilins, in die Synthesestufen des Verfahrens zurückgeführt werden.

Die Erfindung betrifft ein Verfahren zur Herstellung von Di- und Polyaminen der Diphenylmethanreihe (MDA)durch Umsetzung von Anilin und Formaldehyd in Gegenwart eines sauren Katalysators, bei dem
a) Anilin enthaltend weniger als 3 Gew.-%, bevorzugt 0,001 bis 3 Gew.-%, besonders bevorzugt 0,01 bis 1 Gew.-% Di- und Polyamine der Diphenylmethanreihe, bezogen auf das Gewicht des eingesetzten Anilins, mit Formaldehyd in Gegenwart eines sauren Katalysators zu einem Reaktionsgemisch enthaltend Di- und Polyamine umgesetzt wird, und
b) das Reaktionsgemisch enthaltend Di- und Polyamine neutralisiert wird, und
c) das neutralisierte Reaktionsgemisch enthaltend Di- und Polyamine in eine organische Phase enthaltend Di- und Polyamine und eine wässrige Phase aufgetrennt wird, und
d) ggf. die organische Phase mit Wasser gewaschen wird, und
e) aus der organischen Phase überschüssiges Anilin destillativ entfernt wird, und
f) die in den Schritten a) bis e) anfallenden Abwässer und Kondensate teilweise oder vollständig zusammen geführt werden, wobei zumindest die in den Schritten c) und e) erhaltenen Abwässer und Kondensate jeweils zumindest teilweise zusammen geführt werden, und wobei eine Mischung enthaltend Wasser, Di- und Polyamine, Anilin und Salze des in Schritt a) eingesetzten Katalysators erhalten wird, und
g) die in Schritt f) erhaltene Mischung einer Phasentrennung unterzogen wird, wobei ein Anilin enthaltend Di- und Polyamine erhalten wird, und
h) das bei der Phasentrennung erhaltene Anilin enthaltend Di- und Polyamine anschließend zumindest teilweise in die Umsetzung in Schritt a) zurückgeführt wird.

Bevorzugt wird das bei der Phasentrennung in Schritt g) erhaltene Anilin enthaltend Di- und Polyamine zusätzlich teilweise in einen der Schritte b) bis e) eingespeist.

Bevorzugt wird das in Schritt g) gewonnene extrahierte Abwasser einer Extraktion mit Anilin, bevorzugt mit frisch eingesetztem Anilin, unterzogen. Weiterhin bevorzugt werden die dabei erhaltenen Extrakte bevorzugt der in Schritt f) erhaltenen Mischung zugesetzt.

Der zur Erzeugung von Di- und Polyamingemischen mit niedrigen Farbwerten beschriebene Einsatz von Anilin enthaltend MDA in Gehalten von weniger als 3 Gew.-%, bevorzugt 0,001 bis 3 Gew.-%, besonders bevorzugt 0,01 bis 1 Gew.-% MDA, bezogen auf das Gewicht des eingesetzten Anilins, kann in allen bekannten Verfahren zur Herstellung von MDA aus Anilin und Formaldehyd in Gegenwart saurer Katalysatoren erfolgen. Wesentlich ist nur, dass die angegebenen Grenzen des Gehalts an MDA im eingesetzten Anilin in den Apparaten der ein- oder mehrstufigen Umsetzung des Anilins mit Formaldehyd eingehalten werden und örtliche Überkonzentrationen vermieden werden.

Bevorzugt wird das in Schritt a) eingesetzte Anilin durch Vereinigung zumindest einer Teilmenge des in Schritt g) erhaltenen Anilins enthaltend Di- und Polyamine mit aus anderen Quellen stammendem Anilin, bevorzugt frischem Anilin, hergestellt. Dabei ist es wesentlich, dass das eingesetzte Anilin weniger als 3 Gew.-%, bevorzugt 0,001 bis 3 Gew.-%, besonders bevorzugt 0,01 bis 1 Gew.-% MDA enthält. Etwaige Überschüsse des in Schritt g) erhaltenen Anilins enthaltend Di- und Polyamine werden bevorzugt der Neutralisation und/oder Aufbereitung der Di- und Polyamingemische (Schritte b) bis e)) zugeführt.

Diese Verfahrensweise stellt zum einen den für die Herstellung von hellen Di- und Polyamingemischen der Diphenylmethanreihe bzw. den aus ihnen hergestellten Isocyanaten und Polyurethanen notwendigen niedrigen Gehalt an Di- und Polyaminen in dem in der Reaktion in Schritt a) eingesetzten Anilin sicher. Zum anderen minimiert diese Verfahrensweise vorteilhaft den zur Aufbereitung der Polyamingemische in Schritt e) notwendigen Aufwand sowie den zur Aufbereitung der in Schritt g) erhaltenen Extrakte notwendigen Energieeinsatz. Denn die Herstellung der Di und Polyamine der Diphenylmethanreihe erfolgt industriell in einem so großen Maßstab, dass selbst geringe ökonomische Verbesserungen solch wichtiger großtechnischer Verfahren von großem wirtschaftlichen Interesse sind.

Bevorzugt wird in Schritt a) das Anilin enthaltend MDA in Gehalten von weniger als 3 Gew.-%, bevorzugt 0,001 bis 3 Gew.-%, besonders bevorzugt 0,01 bis 1 Gew.-% zunächst mit Formaldehyd in einem Molverhältnis von bevorzugt 1,6:1 bis 10:1, besonders bevorzugt zwischen 1,6: 1 und 4,0:1, und bei einer Temperatur von bevorzugt zwischen 10 und 150°C, besonders bevorzugt zwischen 75 und 110°C, umgesetzt. Eingesetztes Anilin bedeutet dabei das Anilin enthaltend die Di- und Polyamine sowie ggf. weitere Nebenkomponenten.

Dann wird dem Reaktionsgemisch bevorzugt ein saurer Katalysator in einem Molverhältnis Anilin zum sauren Katalysator von bevorzugt 2:1 bis 100:1 1 (entsprechend einem Protonierungsgrad des Anilins von 50 bis 1 %), besonders bevorzugt 4:1 bis 20:1, bei einer Temperatur von bevorzugt 10 bis 150°C, besonders bevorzugt 35 bis 75°C zugegeben. Als saurer Katalysator wird bevorzugt Salzsäure eingesetzt.

Das bei der Kondensation des Formaldehyds mit dem Anilin entstandene und ggf. mit dem Formaldehyd eingetragene Wasser wird dabei bevorzugt ganz oder teilweise vor der Katalysatorzugabe ( z.B. durch Phasentrennung ) oder danach (z.B. durch Siedekühlung und Austrag der dabei gewonnenen Kondensate) abgetrennt.

Das Reaktionsgemisch wird anschließend bevorzugt auf Temperaturen von zwischen 100 bis 180°C, besonders bevorzugt von zwischen 130 bis 160°C, aufgeheizt und nach Erreichen der Endtemperatur bei dieser Temperatur für 5 bis 300 Minuten gehalten.

In Schritt b) wird das Reaktionsgemisch enthaltend MDA anschließend ggf. unter Zusatz von Wasser und / oder Anilin neutralisiert. Bevorzugt erfolgt die Neutralisation mit Natronlauge.

Anschließend wird in Schritt c) das neutralisierte Reaktionsgemisch enthaltend MDA in eine organische Phase enthaltend MDA und eine wässrige Phase aufgetrennt. Dies kann durch die Zugabe von Anilin und / oder Wasser unterstützt werden. Wird die Phasentrennung durch Zugabe von Anilin und / oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und durch Zugabe von Anilin und/oder Wasser verdünnte Reaktionsgemisch wird anschließend bevorzugt einem Apparat zugeführt, der aufgrund seiner Konfiguration und / oder Einbauten besonders zur Auftrennung in eine organische Phase enthaltend MDA und eine wässrige Phase geeignet ist. Zum Einsatz kommen bevorzugt Scheideflaschen mit die Koalescenz der beiden Phasen unterstützenden Plattenpaketen als Einbauten.

In Schritt d) wird ggf. die organische Phase enthaltend MDA bevorzugt bei Temperaturen von zwischen 50 und 150°C, besonders bevorzugt von zwischen 80 und 110°C mit Wasser in einem Verhältnis von Wasser zu organischer Phase von 0,05 bis 2 : 1 gewaschen.

Die in Schritt c) erhaltene organische Phase wird, ggf. nach erfolgter Wäsche in Schritt d), anschließend in Schritt e) in einer Destillation von Anilin gereinigt, wobei ein gereinigtes MDA und ein Kondensat enthaltend Anilin und Wasser erhalten wird.

Die in den Schritten a) bis e) erhaltenen Abwässer, wie die wässrige Phase aus Schritt c), das Waschwasser aus Schritt d) und das Kondensat aus Schritt e) und ggf. weitere Prozesswässer wie beispielsweise weitere kondensierte Brüden oder in Schritt a) erhaltene wässrige Phasen, werden anschließend teilweise oder vollständig in Schritt f) vereinigt. Dabei werden zumindest die in den Schritten c) und e) erhaltenen Abwässer und Kondensate jeweils zumindest teilweise, bevorzugt jeweils zu wenigstens 50%, zusammen geführt. Dabei wird eine Mischung erhalten, welche bevorzugt Wasser sowie 0,001 bis 5 Gew.-% MDA, 0,5 bis 60 Gew.-% Anilin und 1 bis 25 Gew.-% an Salzen des in Schritt a) eingesetzten sauren Katalysators, jeweils bezogen auf das Gewicht der Mischung, enthält.

Die in Schritt f ) erhaltene Mischung wird dann in Schritt g ) bevorzugt bei einer Temperatur von zwischen 30 und 120°C, bevorzugt 70 bis 110°C, einer Phasentrennung unterworfen, wobei ein Anilin enthaltend Di- und Polyamine erhalten wird.

Fakultativ kann das in Schritt g ) erhaltenen Abwasser in einem weiteren Schritt einer Extraktion mit Anilin, bevorzugt mit frisch eingesetztem Anilin, bevorzugt bei einer Temperatur von zwischen 30 und 120°C, besonders bevorzugt 70 bis 110°C, mit Anilin im Gewichts-Verhältnis von Anilin zu Abwasser von bevorzugt 0,05 bis 1: 1, besonders bevorzugt 0,1 bis 0,3:1, extrahiert werden, wobei die Extrakte vorteilhaft der in Schritt f) bereiteten Mischung zugeführt werden. Dabei erfolgt die Extraktion bevorzugt mehrstufig und im Gegenstrom. Bevorzugt wird Anilin als alleiniges Extraktionsmittel eingesetzt.

Vorteilhaft unterstützt wird die Extraktion mit frisch eingesetztem Anilin durch die in Schritt f) durchgeführte Mischung (die mit einer Extraktion einhergeht) der Abwässer mit den Kondensaten der in Schritt e) durchgeführten destillativen Entanilinierung und die in Schritt g) durchgeführte Extraktion. Es sind damit sehr geringe Einsätze an Frischanilin zur Extraktion erforderlich. Dabei werden aufgrund der Phenolfreiheit der Kondensate geringere Phenolbeladungen im extrahierten Abwasser erhalten.

Vorteilhaft ist die destillative Entfernung des Anilins aus dem extrahierten Abwasser. Anilin bildet mit Wasser ein niedrig siedendes Azeotrop, so dass die destillative Entfernung bei einem einfach handhabbarem Unterdruck bei Temperaturen unter 100°C durchgeführt werden kann, dann sog. Abwärmen vorteilhaft als Energien bei der destillativen Rückgewinnung des Anilins eingesetzt werden können.

Die bei der destillativen Anilinentfernung aus dem extrahierten Abwasser erhaltenen Kondensate enthalten einen hohen Wasseranteil. Die Kondensate können deshalb vorteilhaft ganz oder teilweise als Verdünnungswasser in der Neutralisation in Schritt b) und als Waschwasser in Schritt d) eingesetzt werden, besonders bevorzugt erst als Waschwasser in Schritt d), dann als Verdünnungswasser in der Neutralisation in Schritt b). Dabei wird ein großer Waschmitteleinsatz möglich, ohne das Abwasseraufkommen des Verfahrens zu vergrößern.

Werden bei der destillativen Anilinentfernung aus dem extrahierten Abwasser die Brüden mehrstufig kondensiert, kann vorteilhaft eine Fraktion enthaltend Methanol und weitere Leichtsieder in hohen Konzentrationen erzeugt werden. Zum einen wird damit der Methanolpegel in den Verfahrensstufen vorteilhaft abgesenkt, zum anderen kann diese Fraktion vorteilhaft als Brennstoffsubstitut eingesetzt werden.

In Schritt h) wird schließlich das bei der Phasentrennung in Schritt g) erhaltene Anilin enthaltend Di- und Polyamine teilweise oder vollständig in Schritt a) zurückgeführt. Bevorzugt wird dabei das rückgeführte Anilin ohne weitere Aufarbeitung in der Reaktion in Schritt a) eingesetzt. Ggf. wird ein weiterer Teil in einen der Prozessschritte b) bis e) eingesetzt.

Das erfindungsgemäße Verfahren zeichnet sich zum einen durch eine besonders einfache Handhabung der bei der extraktiven Aufarbeitung seiner Abwässer und Kondensate anfallenden anilinhaltigen Ströme aus. Die nach dem erfindungsgemäßen Verfahren hergestellten Polyamine weisen darüber hinaus geringe Gehalte an Chromophoren aus und können vorteilhaft zu gering gefärbten Isocyanaten bzw. hellen Polyurethanprodukten umgesetzt werden. Dabei kann die Phosgenierung der Polyamine sowie die Isolierung der dabei erhaltenen Polyisocyanate und deren Umsetzung zu Polyurethanprodukten gemäß den bekannten technischen Verfahren durchgeführt werden.

Wesentlich an dem erfindungsgemäßen Verfahren ist, dass in der Reaktion von Anilin und Formaldehyd ein Anilin eingesetzt wird, das weniger als 3 Gew.-%, bevorzugt 0,001 bis 3 Gew.-%, besonders bevorzugt 0,01 bis 1 Gew.-% Di- und Polyamine der Diphenylmethanreihe, bezogen auf das Gewicht des eingesetzten Anilins, enthält. Eingesetztes Anilin bedeutet dabei das Anilin enthaltend die Di- und Polyamine sowie ggf. weitere Nebenkomponenten. Durch den geringen Gehalt an Di- und Polyaminen in dem eingesetzten Anilin werden verbesserte Farbwerte der Di- und Polyamine und der daraus hergestellten Di- und Polyisocyanate bzw. Polyurethane erhalten.

Neu und wesentlich an dem erfindungsgemäßen Verfahren ist weiterhin, dass die Extraktion mit Anilin nach der Phasentrennung des neutralisierten Reaktionsgemisches enthaltend MDA erfolgt, dabei nur die Extraktion der wässrigen Phase mit Anilin erfolgt und dazu vorwiegend die in Schritt e) erhaltenen anilinhaltigen Kondensate der destillativen Anilinentfernung als Extraktionsmittel in Schritt g) eingesetzt werden. Denn die Phasentrennung in Schritt g) erfolgt faktisch unter gleichzeitiger Extraktion der wässrigen Phase durch das in den stark anilinhaltigen Kondensaten enthaltene Anilin (organische Phase). Das noch beide Phasen enthaltende Reaktionsgemisch oder die nach der Phasentrennung erhaltene organische Phase wird nach dem erfindungsgemäßen Verfahren nicht mit Anilin extrahiert. Durch die Abtrennung der Abwässer und den Einsatz der Kondensate können im Unterschied zum Stand der Technik der Einsatz und die Aufarbeitung eines zusätzlichen hydrophoben Lösungsmittels entfallen und sind - wenn überhaupt - nur äußerst geringe Mengen an frisch eingesetztem Anilin zur Aufbereitung der Abwässer erforderlich.

Die Färbung der Di- und Polyisocyanate kann durch zwei Absorptionsmaxima im sichtbaren UV-Bereich bei 430 bzw. 520 nm charakterisiert werden. Dabei kann aufgrund entsprechender Erfahrung aus diesen Werten die Färbung der aus den Di- und Polyisocyanaten der Diphenylmethanreihe hergestellten Polyurethanprodukte vorhergesagt werden. Der Wert bei 430 nm entspricht einer gelb-braunen, der Wert bei 520 nm einer grauen Färbung der aus den Di- und Polyisocyanaten hergestellten Polyurethanprodukte. Kleineren Absorptionswerten der Di- und Polyisocyanate entsprechen geringere bzw. hellere Färbungen der aus diesen Di- und Polyisocyanaten hergestellten Polyurthenprodukte.

Die erfindungsgemäßen Verfahren sollen anhand der folgenden Beispiele näher dargestellt werden:

### Beispiele:

### Beispiel 1

### Vergleichsbeispiel mit MDA-freiem technischem Anilin (erfindungsgemäß)

In einem inertisierten Kolben werden von 559 g technischem, bisher nicht in den MDA-Prozess eingesetztem Anilin (6,0 mol) 150 ml Anilin vorgelegt, wird das vorgelegte Anilin unter Rühren auf 80°C erhitzt, werden dann das restliche Anilin zusammen mit 268 g einer 32 gew.-%igen Formalinlösung (2,86 mol) gleichmäßig innerhalb von 20 Minuten zugetropft.

Nach beendeter Zugabe wird das Reaktionsgemisch 5 Minuten bei 80°C nachgerührt, dann unter Luftausschluss in einen inertisierten Scheidekolben überführt, wird in diesem nach einer Separationszeit von 5 Minuten die organische Phase abgetrennt.

In einem inertisierten Kolben werden 150 ml der abgetrennten organischen Phase vorgelegt, dann unter Rühren der Rest der organischen Phase zusammen mit 183 g einer 30 gew.-%igen Salzsäure (1,5 mol) gleichmäßig innerhalb von 30 Minuten zugetropft, dabei die Temperatur durch externe Kühlung auf 35°C gehalten.

Nach beendeter Zugabe der Komponenten wird das Reaktionsgemisch weitere 30 Minuten unter Rühren bei 35°C gehalten, dann innerhalb von 10 Minuten auf 60°C aufgeheizt, wiederum für 30 Minuten bei dieser Temperatur gerührt, dann innerhalb von 30 Minuten auf Rückflusstemperatur aufgeheizt und zur Vervollständigung der Umsetzungen für weitere 10 h unter leichtem Rückfluss gekocht.

Zur Neutralisation des sauren Katalysators werden dem heißen Reaktionsgemisch innerhalb von 5 Minuten 144 g einer 50 gew.-%igen Natronlauge (1,8 mol) zugegeben, wird zur besseren Phasentrennung anschließend das Reaktionsgemisch mit 100 ml siedendem destilliertem Wasser versetzt und für weitere 15 Minuten unter leichtem Rückfluss gehalten.

Die organische Phase wird abgetrennt, zweimal mit Wasser gewaschen, dazu jeweils mit 600 ml siedendem destilliertem Wasser versetzt und für 5 Minuten unter leichtem Rückfluss gehalten, nach der Wäsche in eine Destillationsapparatur überführt und in dieser bei 10 mbar bis zur beginnenden 2-Kerndestillation von anhaftenden Leichtsiedern wie Wasser und überschüssigem Anilin befreit.

Erhalten wird ein Polyamingemisch, gekennzeichnet durch folgende Gehalte
- 4,4'-MDA 54,40 Gew.-%
- 2,4'-MDA 3,27 Gew.-%
- 2,2'-MDA 0,074 Gew.-%
- N-Methyl-MDA 0,39 Gew.-%
- Summe 2-Kern MDA 58,13 Gew.%
- Summe 3-Kern MDA 22,92 Gew.-%
- Summe 4-Kern MDA 10,20 Gew.-%

In einen inertisierten Kolben einer Laborphosgenierapparatur werden
- 310 ml getrocknetes Monochlorbenzol vorgelegt und auf 0°C abgekühlt,
- dann 105 g Phosgen in das abgekühlte Monochlorbenzol eingeleitet und kondensiert, abschließend
- unter intensivem Rühren innerhalb von 10 sec eine 55 grädige Lösung von 50 g des - wie oben skizziert - dargestellten Polyamingemisch in 255 ml Monochlorbenzol zugegeben, wobei sich das Reaktionsgemisch auf ca. 50°C erwärmt.

Die entstandene Suspension wird unter intensivem Rühren und weiterer Phosgenzugabe mit einer Rate von 60 g/h innerhalb von 45 Minuten auf 100°C aufgeheizt, dann innerhalb weiterer 10 Minuten auf Rückfluss gebracht (ca. 135°C) und bis zum Erreichen des Klarpunktes unter Rückfluss erhitzt.

Die Phosgenspeisung wird eingestellt, das klare Reaktionsgemisch in eine Destillationsapparatur verbracht, in dieser bei einem Kopfvakuum von ca. 12 mbar von überschüssigem Phosgen sowie dem Lösungsmittel befreit, anschließend im Feinvakuum (2-3 mbar) bei einer Sumpftemperatur von ca. 195°C für 15 Minuten getempert, dann unter Stickstoffbeschleierung abgekühlt.

Erhalten wird ein MDI-Gemisch, gekennzeichnet durch
- NCO-Gehalt (%) 31,46
- Viskosität / 25°C ( mPas ) 77
- Cl, gesamt (%) 0,16
- E₄₃₀ 0,174 ¹⁾
- E₅₂₀ 0,026 ¹⁾
1) Von dem erhaltenen Isocyanat werden 1,0 g in Chlorbenzol gelöst und mit Chlorbenzol zu 50 ml verdünnt. Die Extinktion der so erhaltenen Lösung wird mit einem Photometer / Dr. Lange LICO 300 bei den beiden Wellenlängen 430 nm und 520 nm bestimmt.

### Beispiel 2

### Umsetzung mit technischem Anilin, dem 2,5 Gew.-% 4,4'-MDA zugesetzt ist (erfindungsgemäß)

Die Umsetzung des vorab mit 2,5 Gew% 4,4'-MDA dotierten technischen Anilins zu den Polyamingemischen sowie die Umsetzung der Polyamingemische zu den entsprechenden Isocyanaten erfolgte analog zu dem Beispiel 1 mit den dort angegebenen Reaktionsbedingungen.

Erhalten wurde ein Polyamingemisch, gekennzeichnet durch folgende Gehalte
- 4,4'-MDA 53,70 Gew.-%
- 2,4'-MDA 2,95 Gew.-%
- 2,2'-MDA 0,054 Gew.-%
- N-Methyl-MDA 0,41 Gew.-%
- Summe 2-Kern MDA 57,11 Gew.-%
- Summe 3-Kern MDA 23,20 Gew.-%
- Summe 4-Kern MDA 10,60 Gew.-%

Aus dem Polyamingemisch wurde ein MDI-Gemisch erhalten, gekennzeichnet durch
- NCO-Gehalt (%) 31,54
- Viskosität / 25°C (mPas) 81
- Cl, gesamt (%) 0,19
- E₄₃₀ 0,212 ¹⁾
- E₅₂₀ 0,037 ¹⁾

### Beispiel 3

### Umsetzung mit technischem Anilin, dem 5,0 Gew.-% 4,4'-MDA zugesetzt ist (nicht erfindungsgemäß)

Die Umsetzung des vorab mit 5,0 Gew.-% 4,4'-MDA dotierten technischen Anilins zu den Polyamingemischen sowie die Umsetzung der Polyamingemische zu den entsprechenden Isocyanaten erfolgte analog zu dem Beispiel 1 mit den dort angegebenen Reaktionsbedingungen.

Erhalten wurde ein Polyamingemisch, gekennzeichnet durch folgende Gehalte
- 4,4'-MDA 52,10 Gew.-%
- 2,4'-MDA 2,64 Gew.-%
- 2,2'-MDA 0,045 Gew.-%
- N-Methyl-MDA 0,41 Gew.-%
- Summe 2-Kern MDA 55,20 Gew.-%
- Summe 3-Kern MDA 23,50 Gew.-%
- Summe 4-Kern MDA 11,20 Gew.-%

Aus dem Polyamingemisch wird ein MDI-Gemisch erhalten, gekennzeichnet durch
- NCO-Gehalt (%) 31,50
- Viskosität / 25°C (mPas) 89
- Cl, gesamt (%) 0,21
- E₄₃₀ 0,266 ¹⁾
- E₅₂₀ 0,05 ¹⁾

### Beispiel 4

### Einsatz von Kondensaten der Entanilinierung sowie frisch eingesetztem Anilin als Extraktionsmittel in einer Abwasseraufbereitung (erfindungsgemäß)

27,4 t/h einer Mischung, enthaltend anteilmäßig Abwässer und Kondensate aus den Stufen eines Verfahrens, dadurch gekennzeichnet, dass
a) Anilin und Formalin in Gegenwart von 30 Gew%iger Salzsäure als Katalysator umgesetzt werden,
b) das Reaktionsgemisch mit Natronlauge neutralisiert wird,
c) die organische Phase abgetrennt wird,
d) die organische Phase mit heißem Wasser gewaschen wird und
e) aus der organischen Phase überschüssiges Anilin destillativ entfernt, die Kondensate ohne weitere Aufarbeitung dem Sammelabwasser (alle Abwässer der Schritte a) - d)) zugeführt werden,
mit einem Gehalt an 0,25 Gew.-% MDA, 25,7 % Anilin und 6,3 Gew.-% Natriumchlorid werden über einen Wärmeaustauscher auf Temperaturen > 80°C aufgeheizt und zu ihrer Aufbereitung einer Separationsstufe (Schritt g)) und nachfolgend einer zweistufigen, im Gegenstrom mit MDA-freiem Anilin als Extraktionsmittel betriebenen Extraktionsstufe zugeführt, wobei
- das mit 1,5 t/h zugeführte MDA-freie Anilin vor seiner Einspeisung in die Separationsstufe (Phasentrennung in Schritt g)) auf Temperaturen > 50°C aufgeheizt wird,
- das frisch eingesetzte wie das die erste und zweite Stufe der Extraktionstufe verlassende Anilin intensiv unter einem Energieeintrag von 0,1kW/m³ Abwasser mit dem im Gegenstrom geführten Abwasser bzw. der eingesetzten Mischung vermischt, dann in den Mixern nachgeschalteten Settlern mit mittleren Verweilzeiten von 20 Minuten unter Einhaltung von Temperaturen > 80°C wieder abgetrennt wird,
- die extrahierten Abwässer zur Entfernung ihres gelösten Anilingehalts einer destillativen Abtrennung zugeführt werden,
- die beladenen Anilinphasen der Separationsstufe (Schritt g)) vollständig in eine Einsatzanilinvorlage gespeist und dort mit frisch bezogenem Anilin vermischt werden,
- dann die Mischung als sogenanntes Einsatzanilin auf die Reaktionsstufen des Verfahrens zurückgeführt wird.

Nach der Extraktion enthält das der nachgeschalteten destillativen Anilinabtrennung zugeführte Abwasser < 1 ppm MDA, ist ebenfalls frei von Aminalen und Aminobenzylaminen. Sein Anilingehalt von 2,1 Gew.-% entspricht dem Lösungsgleichgewicht. Zusätzlich mit der Einsatzmischung der Abwasseraufbereitung zugeführte Anilinmengen bilden mit dem Extrakt die organische Phase der Separationsstufe.

Die organische Phase der Separationsstufe weist MDA-Gehalte von 0,78 Gew.-% auf. Durch Zuspeisung von 10,4 t/h frisch bezogenem technischen Anilin werden MDA-Beladungen von 0,34 % im Einsatzanilin erreicht.

## Patentansprüche

1. Verfahren zur Herstellung von Di- und Polyaminen der Diphenylmethanreihe durch Umsetzung von Anilin und Formaldehyd in Gegenwart eines sauren Katalysators, bei dem
a) Anilin enthaltend weniger als 3 Gew.-% Di- und Polyamine der Diphenylmethanreihe, bezogen auf das Gewicht des eingesetzten Anilins, mit Formaldehyd in Gegenwart eines sauren Katalysators zu einem Reaktionsgemisch enthaltend Di- und Polyamine umgesetzt wird, und
b) das Reaktionsgemisch enthaltend Di- und Polyamine neutralisiert wird, und
c) das neutralisierte Reaktionsgemisch enthaltend Di- und Polyamine in eine organische Phase enthaltend Di- und Polyamine und eine wässrige Phase aufgetrennt wird, und
d) ggf. die organische Phase mit Wasser gewaschen wird, und
e) aus der organischen Phase überschüssiges Anilin destillativ entfernt wird, und
f) die in den Schritten a) - e) anfallenden Abwässer und Kondensate teilweise oder vollständig zusammen geführt werden, wobei zumindest die in den Schritten c) und e) erhaltenen Abwässer und Kondensate jeweils zumindest teilweise zusammen geführt werden, und wobei eine Mischung enthaltend Wasser, Di- und Polyamine, Anilin und Salze des in Schritt a) eingesetzten Katalysators erhalten wird, und
g) die in Schritt f) erhaltene Mischung einer Phasentrennung unterzogen wird, wobei ein Anilin enthaltend Di- und Polyamine erhalten wird, und
h) das bei der Phasentrennung erhaltene Anilin enthaltend Di- und Polyamine anschließend zumindest teilweise in die Umsetzung in Schritt a) zurückgeführt wird.

2. Verfahren nach Anspruchh 1, bei dem in Schritt f) eine Mischung enthaltend Wasser sowie 0,001 bis 5 Gew.-% MDA, 0,5 bis 60 Gew.-% Anilin und 1 bis 25 Gew.-% an Salzen des in Schritt a) eingesetzten sauren Katalysators, jeweils bezogen auf das Gewicht der Mischung, erhalten wird.

3. Verfahren nach Anspruch 1, bei dem die Phasentrennung in Schritt g) bei einer Temperatur von zwischen 30 und 120°C, bevorzugt 70 bis 110°C erfolgt.

4. Verfahren nach Anspruch 1, bei dem das in Schritt g) gewonnene extrahierte Abwasser in einer weiteren Extraktion bei einer Temperatur von zwischen 30 und 120°C, bevorzugt 70 bis 110°C mit Anilin im Gewichts-Verhältnis von Anilin zu Abwasser von 0,05 bis 1:1, bevorzugt 0,1 bis 0,3:1 extrahiert wird und die erhaltenen Extrakte der in Schritt f ) erhaltenen Mischung zugesetzt werden.

5. Verfahren nach Anspruch 4, bei dem in der Extraktion das Abwasser mehrstufig extrahiert wird.

6. Verfahren nach Anspruch 4, bei dem in der Extraktion das Abwasser im Gegenstrom extrahiert wird.

7. Verfahren nach Anspruch 4, bei dem das bei der Extraktion erhaltene Anilin enthaltend MDA teilweise in einen oder mehrere der Schritte a) bis e) zurückgeführt wird.

8. Verfahren nach Anspruch 1, bei dem aus dem bei der Phasentrennung in Schritt g) erhaltenen Abwasser destillativ Anilin entfernt wird und bei dem das ausgetriebene Anilin, bevorzugt in Form seiner kondensierten Brüden, zumindest teilweise in die Neutralisation in Schritt b) und/oder in die Wäsche in Schritt d) zurückgeführt wird.

9. Verfahren nach Anspruch 8, bei dem die bei der destillativen Abtrennung des Anilins erzeugten Brüden mehrstufig kondensiert werden, wobei eine Fraktion enthaltend 20 bis 95 Gew.-%, bevorzugt 60 bis 80 Gew.-%, Methanol erzeugt wird.

10. Verfahren nach Anspruch 7, bei dem aus dem bei der Extraktion erhaltenen Abwasser destillativ Anilin entfernt wird und bei dem das ausgetriebene Anilin, bevorzugt in Form seiner kondensierten Brüden, zumindest teilweise in die Neutralisation in Schritt b) und/oder in die Wäsche in Schritt d) zurückgeführt wird.

11. Verfahren nach Anspruch 10, bei dem die bei der destillativen Abtrennung des Anilin erzeugten Brüden mehrstufig kondensiert werden, wobei eine Fraktion enthaltend 20 bis 95 Gew.-%, bevorzugt 60 bis 80 Gew.-% Methanol erzeugt wird.

## Claims

1. Process for preparing di- and polyamines of the diphenylmethane series by reacting aniline and formaldehyde in the presence of an acidic catalyst, in which
a) aniline containing less than 3% by weight of di- and polyamines of the diphenylmethane series, based on the weight of the aniline used, is reacted with formaldehyde in the presence of an acidic catalyst to give a reaction mixture comprising di- and polyamines, and
b) the reaction mixture comprising di- and polyamines is neutralized, and
c) the neutralized reaction mixture comprising di- and polyamines is separated into an organic phase comprising di- and polyamines and an aqueous phase, and
d) the organic phase is optionally washed with water, and
e) excess aniline is removed by distillation from the organic phase, and
f) some or all of the wastewater and condensates obtained in steps a) - e) are combined, at least the wastewater and condensates obtained in step c) and e) being at least partly combined in each case, to obtain a mixture comprising water, di- and polyamines, aniline and salts of the catalyst used in step a), and
g) the mixture obtained in step f) is subjected to a phase separation to obtain an aniline comprising di- and polyamines, and
h) the aniline comprising di- and polyamines obtained in the phase separation is subsequently at least partly recycled into the reaction in step a).

2. Process according to Claim 1, in which a mixture comprising water and 0.001 to 5% by weight of MDA, 0.5 to 60% by weight of aniline and 1 to 25% by weight of salts of the acidic catalyst used in step a), based in each case on the weight of the mixture, is obtained in step f).

3. Process according to Claim 1, in which the phase separation in step g) is effected at a temperature of between 30 and 120°C, preferably 70 to 110°C.

4. Process according to Claim 1, in which the extracted wastewater obtained in step g) is extracted in a further extraction at a temperature of between 30 and 120°C, preferably 70 to 110°C, with aniline in a weight ratio of aniline to wastewater of 0.05 to 1:1, preferably 0.1 to 0.3:1, and the extracts obtained are added to the mixture obtained in step f).

5. Process according to Claim 4, in which the extraction of the wastewater is a multistage extraction.

6. Process according to Claim 4, in which the extraction of the wastewater is a countercurrent extraction.

7. Process according to Claim 4, in which the aniline comprising MDA obtained in the extraction is recycled partly into one or more of steps a) to e) .

8. Process according to Claim 1, in which aniline is removed by distillation from the wastewater obtained in the phase separation in step g), and in which the aniline driven out, preferably in the form of condensed vapours thereof, is recycled at least partly into the neutralization in step b) and/or into the washing in step d).

9. Process according to Claim 8, in which the vapours obtained in the distillative removal of the aniline are subjected to multistage condensation to obtain a fraction containing 20 to 95% by weight, preferably 60 to 80% by weight, of methanol.

10. Process according to Claim 7, in which aniline is removed by distillation from the wastewater obtained in the extraction, and in which the aniline driven out, preferably in the form of condensed vapours thereof, is recycled at least partly into the neutralization in step b) and/or into the washing in step d).

11. Process according to Claim 10, in which the vapours obtained in the distillative removal of the aniline are subjected to multistage condensation to obtain a fraction containing 20 to 95% by weight, preferably 60 to 80% by weight, of methanol.

## Revendications

1. Procédé de fabrication de di- et polyamines de la série du diphénylméthane par réaction d'aniline et de formaldéhyde en présence d'un catalyseur acide, selon lequel
a) de l'aniline contenant moins de 3 % en poids de di- et polyamines de la série du diphénylméthane, par rapport au poids de l'aniline utilisée, est mise en réaction avec du formaldéhyde en présence d'un catalyseur acide pour former un mélange réactionnel contenant des di- et polyamines, et
b) le mélange réactionnel contenant des di- et polyamines est neutralisé, et
c) le mélange réactionnel contenant des di- et polyamines neutralisé est séparé en une phase organique contenant des di- et polyamines et une phase aqueuse, et
d) la phase organique est éventuellement lavée avec de l'eau, et
e) l'aniline en excès est éliminée par distillation de la phase organique, et
f) les eaux résiduaires et les condensats formés lors des étapes a) à e) sont rassemblés en partie ou en totalité, au moins les eaux résiduaires et les condensats obtenus lors des étapes c) et e) étant à chaque fois rassemblés au moins en partie, et un mélange contenant de l'eau, des di- et polyamines, de l'aniline et des sels du catalyseur utilisé à l'étape a) étant obtenu, et
g) le mélange obtenu à l'étape f) est soumis à une séparation de phases, une aniline contenant des di- et polyamines étant obtenue, et
h) l'aniline contenant des di- et polyamines obtenue lors de la séparation de phases est ensuite recyclée au moins en partie dans la réaction de l'étape a).

2. Procédé selon la revendication 1, selon lequel un mélange contenant de l'eau, ainsi que 0,001 à 5 % en poids de MDA, 0,5 à 60 % en poids d'aniline et 1 à 25 % en poids de sels du catalyseur acide utilisé à l'étape a), à chaque fois par rapport au poids du mélange, est obtenu à l'étape f).

3. Procédé selon la revendication 1, selon lequel la séparation de phases de l'étape g) a lieu à une température comprise entre 30 et 120 °C, de préférence de 70 à 110 °C.

4. Procédé selon la revendication 1, selon lequel l'eau résiduaire extraite obtenue à l'étape g) est extraite lors d'une extraction supplémentaire à une température comprise entre 30 et 120 °C, de préférence de 70 à 110 °C, avec de l'aniline en un rapport en poids entre l'aniline et l'eau résiduaire de 0,05 à 1:1, de préférence de 0,1 à 0,3:1, et les extraits obtenus sont ajoutés au mélange obtenu à l'étape f).

5. Procédé selon la revendication 4, selon lequel l'extraction de l'eau résiduaire est réalisée en plusieurs étapes.

6. Procédé selon la revendication 4, selon lequel l'extraction de l'eau résiduaire est réalisée à contre-courant.

7. Procédé selon la revendication 4, selon lequel l'aniline contenant du MDA obtenue lors de l'extraction est recyclée en partie dans une ou plusieurs des étapes a) à e) .

8. Procédé selon la revendication 1, selon lequel l'aniline est éliminée par distillation de l'eau résiduaire obtenue lors de la séparation de phases à l'étape g) et selon lequel l'aniline éliminée, de préférence sous la forme de ses vapeurs condensées, est recyclée au moins en partie dans la neutralisation de l'étape b) et/ou dans le lavage de l'étape d).

9. Procédé selon la revendication 8, selon lequel les vapeurs formées lors de la séparation par distillation de l'aniline sont condensées en plusieurs étapes, une fraction contenant 20 à 95 % en poids, de préférence 60 à 80 % en poids, de méthanol étant formée.

10. Procédé selon la revendication 7, selon lequel l'aniline est éliminée par distillation de l'eau résiduaire obtenue lors de l'extraction et selon lequel l'aniline éliminée, de préférence sous la forme de ses vapeurs condensées, est recyclée au moins en partie dans la neutralisation de l'étape b) et/ou dans le lavage de l'étape d).

11. Procédé selon la revendication 10, selon lequel les vapeurs formées lors de la séparation par distillation de l'aniline sont condensées en plusieurs étapes, une fraction contenant 20 à 95 % en poids, de préférence 60 à 80 % en poids, de méthanol étant formée.
